# FASCICULE DE BREVET EUROPEEN

(11) **EP 0 610 146 B1**
(45) Date de publication et mention de la délivrance du brevet: **22.07.1998**
(21) Numéro de dépôt: 94420039.3
(22) Date de dépôt: 03.02.1994
(51) Int. Cl.: A61F 2/34

(54) **Prothèse cotyloidienne expansible**
Dehnbare Hüftpfannenprothese
Expansible cotyloid prosthesis

(30) Priorité: 04.02.1993 FR 9301437
(43) Date de publication de la demande: 10.08.1994
(73) Titulaire: Chauvin, Jean-Luc, 84100 Orange (FR)
(72) Inventeur: Chauvin, Jean-Luc, 84100 Orange (FR)
(74) Mandataire: Somnier, Jean-Louis

(56) Documents cités:
- EP-A- 0 277 511
- EP-A- 0 486 403
- FR-A- 2 645 433
- US-A- 4 524 467

## Description

La présente invention a trait à une prothèse cotyloïdienne expansible comportant trois éléments pour le remplacement du cotyle de l'os iliaque lorsque ce dernier est endommagé selon le préambule de la revendication 1.

On connaît des prothèses cotyloïdiennes de ce genre telles que celles décrites dans la demande de brevet EP 486403 et qui comprennent un cotyle artificiel pourvu de fentes d'expansion qui sont symétriquement et régulièrement réparties sur sa périphérie et qui déterminent des segments percés de trous débouchants. Ces prothèses comprennent encore une bague intermédiaire filetée qui est destinée à être vissée dans le cotyle. Cette bague permet la mise en place d'un insert de frottement dans lequel vient s'emboîter une boule céphalique humaine ou artificielle.

De telles prothèses comportent certains inconvénients en qui concerne le maintien du cotyle artificiel dans la cavité de l'os iliaque. En effet, ce cotyle est retenu à l'aide de vis qui coopèrent avec les alésages ménagés entre chaque fente d'expansion. La solidarisation des segments prévus entre chaque fente rend le cotyle complètement rigide, ce qui a pour effet de contraindre la cavité de l'os iliaque.

C'est à ces inconvénients qu'entend plus spécialement remédier la présente invention.

La prothèse cotyloïdienne suivant la présente invention comprend en combinaison :
- un cotyle artificiel ou calotte hémisphérique creuse qui comporte un certain nombre de fentes d'expansion régulièrement réparties sur sa périphérie, un élément intermédiaire fileté qui présente un diamètre extérieur supérieur au diamètre interne du cotyle et un insert en matière plastique tel que du polyéthylène et qui comprend une rainure, lequel élément intermédiaire sur sa face interne et lequel insert sur sa face externe comportent des bossages et de encoches coopérant ensembles pour leur positionnement et leur mise ne place angulaire ; ces éléments composant la dite prothèse cotyloïdienne sont tels que :
   le cotyle artificiel comporte un appendice fileté prévu sur sa face externe et plus particulièrement en son pôle et un trou taraudé qui débouche à l'intérieur du cotyle et au centre de l'appendice ;
- l'élément intermédiaire est un noyau ayant un profil extérieur correspondant à celui interne du cotyle, et comporte un doigt fileté qui coopère avec le trou taraudé du cotyle et sur sa face interne une série d'encoches qui sont régulièrement réparties sur sa périphérie ;
- et l'insert en matière plastique comprend un certain nombre de dents qui coopèrent avec des encoches du noyau intermédiaire et en dessous des dents la rainure est bordée par un bossage.

Le dessin annexé, donné à titre d'exemple, permettra de mieux comprendre l'invention, les caractéristiques qu'elle présente et les avantages qu'elle est susceptible de procurer.

Fig. 1 est une vue éclatée en perspective illustrant les divers composants de la prothèse cotyloïdienne suivant la présente invention.

Fig. 2 est une coupe transversale montrant la prothèse cotyloïdienne introduite dans la cavité de l'os iliaque d'un patient.

Fig. 3 est une vue de détail de mise ne place de l'insert à l'intérieur du noyau intermédiaire.

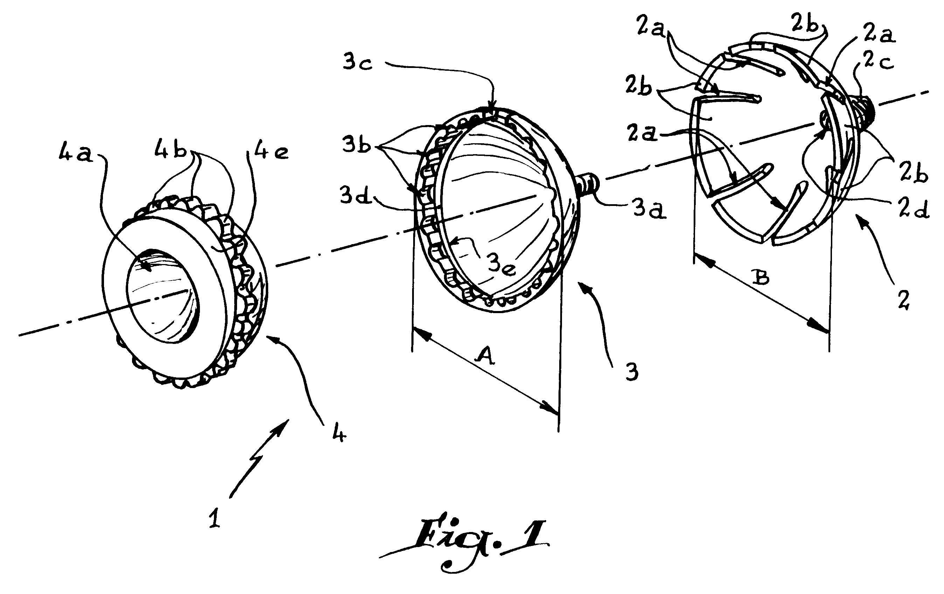

On a représenté en figure 1 une prothèse cotyloïdienne expansible 1, comportant un cotyle artificiel 2 dans lequel vient se visser un noyau intermédiaire 3 qui reçoit un insert 4 en matière plastique telle que du polyéthylène; le cotyle artificiel 2 et le noyau intermédiaire 3 sont préférablement réalisés dans une matière bio-résorbable ou métallique telle que du titane.

Le cotyle artificiel ou calotte creuse 2 comporte un certain nombre de fentes d'expansion 2a qui sont régulièrement et symétriquement réparties sur sa périphérie. Les fentes 2a sont ménagées en direction du pôle du cotyle de manière à former entre chaque fentes un segment 2b. Au niveau du pôle du cotyle 2 et sur sa face externe est prévu un appendice fileté 2c tourné de manière centrifuge. L'appendice fileté 2c est percé en son centre d'un trou taraudé 2d qui débouche à l'intérieur du cotyle 2.

Le noyau intermédiaire 3 est prévu d'un profil extérieur correspondant à celui interne du cotyle 2, mais avec un diamètre extérieur A supérieur au diamètre interne B dudit cotyle.

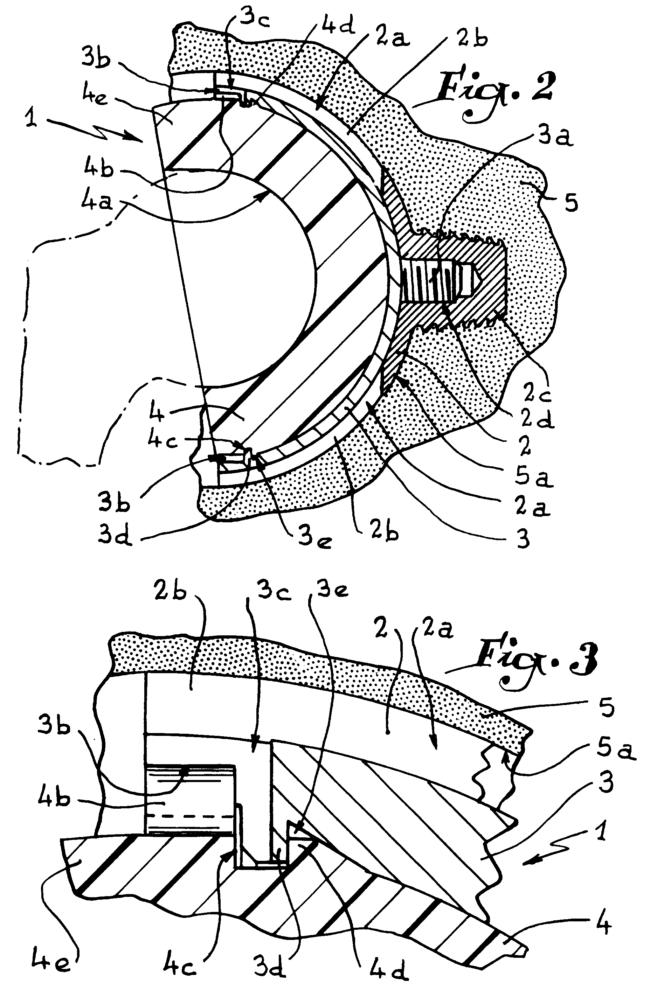

Au niveau du pôle du noyau intermédiaire 3 et sur sa face externe est prévu un doigt fileté 3a tourné vers l'extérieur et qui coopère avec le trou taraudé 2d du cotyle au moment de sa fixation. La face interne du noyau 3 est pourvue d'une série d'encoches 3b qui sont réparties régulièrement sur sa périphérie. La série d'encoches 3b est interrompue par une ouverture 3c dont on verra mieux plus loin la fonction. En dessous des encoches 3b est prévu un jonc 3d qui est bordé dans sa partie inférieure par une rainure 3e en forme de coin, comme représenté en fig. 2 et 3.

L'insert 4 réalisé en matière plastique comporte en son milieu un cavité 4a qui permet de recevoir une boule céphalique artificielle ou humaine représentée en traits mixtes en fig. 2. L'insert 4 comprend sur sa face externe des dents 4b qui sont régulièrement réparties sur sa périphérie. Les dents 4b présentent un profil externe identique au jeu près à celui des encoches 3b qui sont ménagées dans le noyau intermédiaire 3. En dessous des dents 4b est usinée une rainure 4c qui est bordée par un cordon 4d. Au-dessus des dents 4b, l'insert 4 comporte un talon 4e à profil incliné permettant d'éviter la luxation de la boule céphalique lorsque celle-ci est contrainte par des efforts importants.

L'opérateur doit procéder de la manière suivante pour la mise en place à l'intérieur d'une cavité humaine 5a de l'os iliaque 5 d'une prothèse cotyloïdienne décrite précédemment :
- il visse le cotyle 2 à l'aide de l'appendice 2c à l'intérieur de la cavité 5a de manière qu'il vienne en butée contre le fond,
- il visse le noyau intermédiaire 3 à l'intérieur du cotyle 2 de manière a venir écarter ce dernier contre l'intérieur de la cavité 5a. Cet écartement est réalisé par la différence de diamètre entre le noyau intermédiaire 3 et le cotyle 2, ce qui permet une fixation souple et sans contrainte,
- il introduit l'insert plastique 4 à l'intérieur du noyau intermédiaire 3,
- il règle angulairement l'insert 4 dans le noyau intermédiaire 3 au moyen des dents 4b et des encoches 3b,
- il fixe l'insert 4 en appuyant sur ce dernier de manière que coopèrent d'une part sa rainure 4c avec le jonc 3d et d'autre part son cordon 4d avec la rainure 3e.

Lorsque l'opérateur doit retirer l'insert plastique 4, il introduit dans l'ouverture 3c du noyau intermédiaire 3 un outil pour venir prendre appui en dessous des dents 4b afin de faire levier.

## Revendications

1. Prothèse cotyloïdienne comprenant un cotyle artificiel ou calotte hémisphérique creuse (1) en métal qui comporte un certain nombre de fentes d'expansion (2a) régulièrement réparties sur sa périphérie, un élément intermédiaire fileté (3) qui présente un diamètre extérieur (A) supérieur au diamètre (B) du cotyle, et un insert (4) en matière plastique telle que du polyéthylène et qui comprend une rainure (4c) lequel élément intermédiaire (3) sur sa face interne et lequel insert (4) sur sa face externe comportent des bossages et des encoches coopérant ensembles pour leur positionnement et leur mise en place angulaire, caractérisée en ce que :
- ledit cotyle artificiel (2) comporte un appendice fileté (2c) prévu sur sa face externe, plus particulièrement en son pôle et un trou taraudé (2d) qui débouche à l'intérieur du cotyle (2) et au centre de l'appendice (2c) ;
- l'élément intermédiaire est un noyau (3) ayant un profil extérieur correspondant à celui interne du cotyle (2), et comporte un doigt fileté (3) qui coopère avec le trou taraudé (2d) du cotyle (2) et sur sa face interne une série d'encoches (3b) qui sont régulièrement réparties sur sa périphérie ;
- et l'insert en matière plastique (4) comprend un certain nombre de dents (4b) qui coopèrent avec des encoches (3b) du noyau intermédiaire et en dessous des dents la rainure (4c) est bordée par un bossage (4d).

2. Prothèse cotyloïdienne suivant la revendication 1, caractérisée en ce que les fentes d'expansion (2a) sont toutes dirigées en direction de pôle du cotyle (2) de manière à conformer ce dernier en plusieurs segments (2b) de même surface.

3. Cotyle suivant la revendication 1, caractérisé en ce que le noyau intermédiaire (3) comporte sur sa face interne et en dessous des encoches (3b) un jonc (3d) qui est bordé dans sa partie inférieure par une rainure en forme de coin (3e).

4. Cotyle suivant la revendication 1, caractérisé en ce que le noyau intermédiaire (3) comporte sur sa périphérie une ouverture (3c) qui interrompt les encoches (3b).

5. Prothèse suivant la revendication 1, caractérisée en ce que le cotyle artificiel (2) et le noyau intermédiaire (3) sont réalisés dans une même matière telle que du titane.

## Claims

1. An acetabulum prosthesis comprising an artificial acetabulum or hollow hemispherical cup (1) made of metal, which includes a certain number of expansion slots (2a) regularly distributed around its periphery, a threaded intermediate element (3) having an outside diameter (A) greater than the diameter (B) of the acetabulum, and an insert (4) of plastics material such as polyethylene and including a groove (4c), with the intermediate element (3) on its inner face and the insert (4) on its outer face including projections and indentations that co-operate together for the purpose of positioning them and setting their angular position, the prosthesis being characterized in that:
• said artificial acetabulum (2) includes a threaded appendix (2c) provided on its outer face, more particularly at its pole, together with a tapped hole (2d) opening out to the inside of the acetabulum (2) and in the center of the appendix (2c);
• the intermediate element is a core (3) having an outside profile corresponding to the inside profile of the acetabulum (2), and including a threaded finger (3) that co-operates with the tapped hole (2d) in the acetabulum (2), and on its inside face a series of notches (3b) which are regularly distributed around its periphery; and
• the insert of plastics material (4) comprises a certain number of teeth (4b) which co-operate with the notches (3b) of the intermediate core and beneath the teeth, the groove (4c) is bordered by a projection (4d).

2. An acetabulum prosthesis according to claim 1, characterized in that the expansion slots (2a) are all directly towards the pole of the acetabulum (2) so as to cause it to be in the form of a plurality of same-area segments (2b).

3. An acetabulum according to claim 1, characterized in that the intermediate core (3) includes on its inside face and beneath the notches (3b), a retaining ring (3d) which is bordered in its bottom portion by a wedge-shaped groove (3e).

4. An acetabulum according to claim 1, characterized in that the intermediate core (3) includes on its periphery, an opening (3c) which interrupts the notches (3b).

5. A prosthesis according to claim 1, characterized in that the artificial acetabulum (2) and the intermediate core (3) are made of the same material, such a titanium.

## Patentansprüche

1. Gelenkpfannenprothese (1) mit einer künstlichen Gelenkpfanne oder einer halbkugelförmigen hohlen Kalotte aus Metall, die eine bestimmte Anzahl von gleichmäßig auf ihrem Umfang verteilten Dehnungsspalten (2a), ein mit Außengewinde versehenes Zwischenelement (3) mit einem Außendurchmesser (A), der größer als der Durchmesser (B) der Gelenkpfanne ist, und einen Einsatz (4) aus Kunststoff, zum Beispiel Polyethylen, mit einer Rille (4c) umfaßt, wobei das Zwischenelement (3) auf seiner Innenseite und der Einsatz (4) auf seiner Außenseite buckelartige Vorsprünge und Vertiefungen aufweisen, die zu ihrer Positionierung und ihrer winkelmäßigen Festlegung zusammenwirken, dadurch gekennzeichnet, daß
- die künstliche Gelenkpfanne (2) einen mit Außengewinde versehenen Ansatz (2c) an ihrer Außenseite, insbesondere an ihrem Pol, sowie ein Gewindeloch (2d) aufweist, das im Inneren der Gelenkpfanne (2) und im Zentrum des Ansatzes (2c) mündet,
- das Zwischenelement ein Kernteil (3) ist, dessen Außenprofil dem Innenprofil der Gelenkpfanne (2) entspricht und das einen mit Außengewinde versehenen Zapfen (3a), der mit dem Gewindeloch (2d) der Gelenkpfanne (2) zusammenwirkt, sowie eine Reihe von gleichmäßig auf seinem Umfang verteilten Vertiefungen (3b) auf seiner Innenseite aufweist
- und der Einsatz (4) aus Kunststoff eine bestimmte Anzahl von buckelartigen Vorsprüngen (4b) aufweist, die mit den Vertiefungen (3b) des als Zwischenelement dienenden Kernteils zusammenwirken, und die Rille (4c) unterhalb der buckelartigen Vorsprünge am Rand einen Vorsprung (4d) aufweist.

2. Gelenkpfannenprothese nach Anspruch 1, dadurch gekennzeichnet, daß die Dehnungsspalten (2a) sämtlich zum Pol der Gelenkpfanne (2) hin gerichtet und so angeordnet sind, daß sie diese in mehrere Segmente (2b) gleicher Fläche teilen.

3. Gelenkpfannenprothese nach Anspruch 1, dadurch gekennzeichnet, daß das als Zwischenelement dienende Kernteil (3) an seiner Innenseite und unterhalb der Vertiefungen (3b) einen ringförmigen Vorsprung (3d) aufweist, an den sich innenseitig eine keilförmige Rille (3e) anschließt.

4. Gelenkpfannenprothese nach Anspruch 1, dadurch gekennzeichnet, daß das als Zwischenelement dienende Kernteil (3) an seinem Umfang eine Öffnung (3c) aufweist, die die Vertiefungen (3b) unterbricht.

5. Gelenkpfannenprothese nach Anspruch 1, dadurch gekennzeichnet, daß die künstliche Gelenkpfanne (2) und das als Zwischenelement dienende Kernteil (3) aus dem gleichen Material, zum Beispiel Titan, hergestellt sind.
